# EUROPEAN PATENT SPECIFICATION

(11) **EP 2 276 446 B1**
(45) Date of publication and mention of the grant of the patent: **20.02.2013**
(21) Application number: 09741896.6
(22) Date of filing: 07.05.2009
(51) Int. Cl.: A61K 6/10, A61K 6/00, A61K 6/083

(54) **RADICAL POLYMERISATION INITIATORS FOR LIGHT-CURABLE DENTAL MATERIALS**
RADIKALPOLYMERISATIONSINITIATOREN FÜR LICHTHÄRTBARES DENTALMATERIAL
INITIATEURS DE POLYMÉRISATION RADICALAIRE POUR MATÉRIAUX DENTAIRES DURCISSABLES À LA LUMIÈRE

(30) Priority: 08.05.2008 EP 08008693
(43) Date of publication of application: 26.01.2011
(73) Proprietor: Dentsply DeTrey GmbH, 78467 Konstanz (DE)
(72) Inventor: BLACKWELL, Gordon, 78465 Konstanz (DE)
(74) Representative: Hartz, Nikolai
(86) International application number: PCT/EP2009/003278
(87) International publication number: WO 2009/135677

(56) References cited:
- WO-A-02/085974
- US-A- 4 162 162

## Description

### Field of the invention

The present invention relates to light-curable dental materials, such as dental composites and dental adhesives, comprising at least one polymerizable compound having at least one ethylenically unsaturated bond, a hexaaryl bisimidazole, e.g. as a photopolymerization initiator and a co-initiator selected from the following compound classes: thiols, heteroaromatic thiols, benzothiazoles, benzooxazoles, tertiary amines, alcohols, and thiocarboxylic acids. The invention further relates to the use of a hexaarylbisimidazole polymerization initiator in light-curable dental materials. The invention also relates to cured dental materials obtainable by light-curing the light-curable dental materials of the invention.

### Background of the invention

Radical polymerisation of ethylenically unsaturated compounds is widely used to harden dental materials such as dental filling materials. In light-curable dental materials, the ethylenically unsaturated compounds are activated to be polymerisable by the application of light, since this allows a "command cure". By this means the ethylenically unsaturated substances remain workable for an indefinite time, but can be cured at will in a short time by the application of light. Generally the actinic light has a wavelength between about 200 nm and 700 nm, and more often between about 300 nm and 600 nm. In light-curable dental compositions, camphor quinone which allows photopolymerization with visible light is almost exclusively used in combination with an amine as a reductant (co-initiator).

In many cases, it is necessary or convenient to apply the activated dental material or to model it under ambient light. Very often a relatively strong ambient light is needed so that the modelling or application can be carried out with the necessary precision, which leads to several conflicts. If sufficient ambient light is present for accurate and precise use of the dental material, the life time of the activated material is reduced, and the time available for the modelling or application is also reduced. If less ambient light is present, the life time of the material and the time available to model it ("working time") is increased, but the precision of the modelling is reduced. The lifetime of the material towards ambient light may be increased by, for instance, reduction in the concentration of initiators, or increasing the amount of polymerisation inhibitor present However, both of these measures can lead to a decrease in the physical properties of the light-cured dental material.

It is therefore an object of the invention to provide a light-curable dental material having a longer working time under ambient light without compromising the physical properties of the cured dental material.

### Summary of the invention

The invention provides a light-curable dental material comprising (i) a polymerizable compound having at least one ethylenicaily unsaturated bond, (ii) a hexaaryl bisimidazole, and (iii) a co-initiator selected from the following compound classes: thiols, heteroaromatic th lois, benzothiazoles, benzooxazoles, tertiary amines, alcohols, and thiocarboxylic acids.

The hexaaryl bisimidazole may be used as a polymerization initiator for polymerizing said polymerizable compound. The invention also provides a light-curable dental material comprising a hexaaryl bisimidazole (e.g. as a polymerization initiator or inhibitor) and a second compound capable of acting as a polymerization initiator. Said second compound may be an α,β-diketone such as camphor quinone or acetophenone, a phosphine oxide, or a compound containing a single carbonyl group such as benzophenone, benzoin, or benzoin methylether.

The invention further provides a light-cured dental material obtained or obtainable by light curing the light-curable dental materials of the invention. Further, a use of a hexaaryl bisimidazole in a light-curable dental material is provided, notably a use of a hexaaryl bisimidazole as a photopolymerization initiator.

In the present invention, it has surprisingly been found that longer working times can be obtained using hexaaryl bisimidazole as a polymerization initiator in light-curable dental materials compared to prior art light-curable dental materials that use almost exclusively the camphor quinone/amine initiator system. Even more surprisingly, the physical properties of the light-cured dental materials are not compromized to a significant extent. Generally, the physical properties of the light-cured dental materials are even improved compared to light-curable dental materials that use the camphor quinone/amine initiator system. Further, it has been found that the working times of conventional light-curable dental materials containing the camphor quinone/amine initiator system can be extended if the hexaaryl bisimidazole of the invention is additionally included.

The hexaaryl bisimidazole used as a polymerization initiator in the present invention has the general structure of formula (I)

Ar₃T-TAr₃ (I)

wherein each T represents an imidazole moiety and each Ar stands for an optionally substituted aryl group. Multiple Ar in compounds of formula (I) may be the same or different. The substitution on different groups Ar may be the same or different. The aryl groups in the hexaaryl bisimidazole compounds used in the present invention may be substituted or unsubstituted phenyl, naphthyl, phenanthryl, 2-pyridyl, 3-pyridyl, 4-pyridyl, or quinolinyl groups. Substituted or unsubstituted phenyl, naphthyl, or phenanthryl groups are preferred, and substituted or unsubstituted phenyl and naphthyl groups are most often used. In one embodiment, all aryl groups of the hexaaryl bisimidazole compounds are substituted or unsubstituted phenyl groups. The optional substituents of groups Ar are as described below with regard to Ar¹, Ar², and Ar³.

The hexaaryl bisimidazole may be a homodimer or a heterodimer obtainable by oxidation of any of the triarylimidazoles represented by the following formula (II):

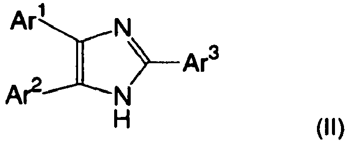

In formula (II), Ar¹, Ar², and Ar³ are aryl groups as defined above. Ar¹, Ar², and Ar³ may be the same or different and each represents an optionally substituted aryl group. Multiple Ar¹, Ar², or Ar³ on the same hexaarylbisimidazole may be the same or different. In one embodiment, Ar¹, Ar² are optionally substituted phenyl groups and Ar³ is an optionally substituted phenyl or naphthyl group.

Each substituted aryl group may have 1 to 5, preferably 1 to 3, substituents selected from the group consisting of C₁₋₆-alkyl, C₁₋₆-alkoxy, hydroxy, halo, cyano, nitro, nitroso, mercapto, carboxyl, sulfonate, thiol, amino, phenyl, pyridyl, and trifluoromethyl. The C₁₋₆-alkyl groups and the C₁₋₆-alkyl groups of the C₁₋₆-alkoxy groups may by linear or branched C₁₋₆-alkyl groups or C₃₋₆ cyclic alkyl groups. Examples of C₁₋₆-alkyl groups are methyl, ethyl, propyl (such as i-propyl or n-propyl), butyl (such as n-butyl, isobutyl, tert-butyl or sec-butyl), pentyl, and hexyl. "Halo" stands for the class consisting of fluoro, chloro, bromo or iodo.

The hexaarylbisimidazole compounds can be prepared as known in the art by oxidation of triarylimidazole compounds. A procedure for the preparation of hexaarylbisimidazoles is described for example in DE 1470 154 and in US 4,459,349.

The structure of the hexaarylbisimidazole compounds to be used in the present invention may be represented by the following formula (III): 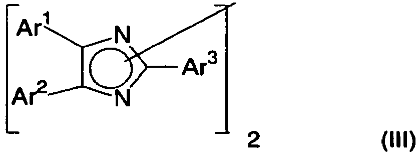 wherein Ar¹, Ar² and Ar³ are as defined above and multiple Ar¹, Ar² or Ar³ on the same hexaarylbisimidazole compound may be the same or different. There are various possibilities for the bonding between the two imidazole moieties. Different atoms within each imidazole moiety may be involved in this bond. This bond may be between two imidiazolo nitrogen atoms, between a nitrogen atom of one imidazolo moiety and a carbon atom of the other imidazolo moiety, or between two carbon atoms of the imidazolo moieties. The general structure of formula (III) is shown with the two moieties joined by a line. In fact, two or more alternatively linked hexaarylbisimidazole compounds may co-exist e.g. in an equilibrium.

An example of a hexaarylbisimidazole compound usable in the invention is shown in formula (IV), known as 2,2'-bis(o-chlorophenyl)-4,4',5,5'-tetraphenyl-1,2'-biimidazole, which is also identified by the abbreviations BCIM, o-Cl HABI or 2-Cl HABI. HABI stands for "hexaarylbisimidazole".

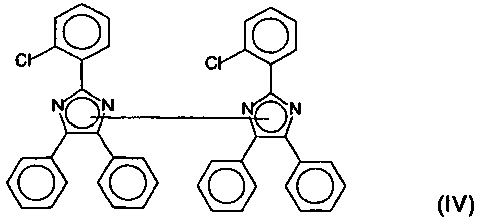

A further example of a commercially available HABI compound is shown in the following formula (V), and illustrates the possibility of obtaining a HABI structure with different substituents on each imidazole moiety. The compound of formula (V) is known as 2,2',4-tris(2-chlorophenyl)-5-(3,4-dimethoxyphenyl)-4',5'-diphenyl-1,1'-biimidazole, abbreviated to TCDM-HABI or CZ-HABI by various manufacturers. In fact this HABI probably exists as an equilibrium mixture of the compounds of the compound class shown, together with homo-dimers of the two imidazole structures.

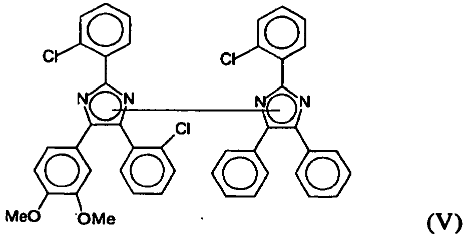

By adjusting the nature of the aryl groups and their substituents, the properties and stability of the hexaarylbisimidazole and the resulting radicals can be adjusted. The stability of the radicals produced by splitting the hexaarylbisimidazole into two triarylimidazole radicals can be estimated by measuring the time needed for the colour of the radical to fade, since this is related to the time needed for the two moieties to recombine or be otherwise annihilated. The less stable a radical is, the higher is its fading rate and the shorter is its lifetime. The values for the "fading rate" given in Table 1 are obtained from US 3,784,557, and were measured by first illuminating a solution of the bisimidazole in benzene, and then measuring the absorption of the solution at the absorption maximum at suitable minute intervals. A plot of 1/(abs - abs∞) against time gives a graph, the slope of which is the "fading rate", where abs is the absorption of the solution at time t and abs∞ is the absorption of the solution after leaving it in the dark over night. The absorption maximum and the extinction coefficient can be adjusted by altering the type and position of the substituents, as is illustrated by values drawn from the literature for a range of substituents, and shown in. Obviously, further combinations and variations of the substituents are possible, and those given earlier or shown in Table 1 are for illustrative purposes only.

The substituents defined in Table 1 relate to the homodimer of formula (VI) below. Numbers in Table 1 indicate the position of the indicated substituent. "0" indicates that the indicated substituent is not present. "2-phenyl substituent 1" relates to the 2-phenyl group on both triarylimidazole moieties. "2-phenyl substituent 2" relates to a possible second substituent on the 2-phenyl group on both triarylimidazole moietes. In all cases of Table 1, substituents P" and "Q" are present in both triarylimidazole moieties of the hexaarylbisimidazole of formula (VI).

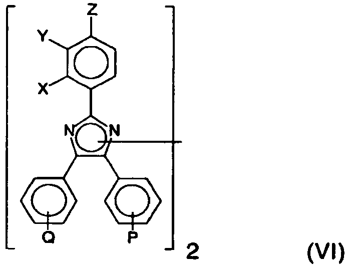

**Table 1**

| substituent | 2-phenyl substituent 1 position X, Y, or Z | 2-phenyl substituent 2 position X, Y, or Z | 4-phenyl substituent position P | 5-phenyl substituent position Q | fading rate abs⁻¹. min⁻¹ | radical absorption max (nm) | extinction coefficient |
|---|---|---|---|---|---|---|---|
| methoxy | 4 | 0 | 4 | 4 | 0.0077 | | |
| methoxy | 4 | 3 | 0 | 0 | 0.091 | | |
| methoxy | 4 | 0 | 0 | 0 | 0.092 | 610 | 6.9 |
| chloro | 0 | 0 | 2 | 0 | 0.2 | 530 | 2.94 |
| methyl | 4 | 0 | 0 | 0 | 0.22 | | |
| bromo | 4 | 0 | 0 | 0 | 0.36 | 560 | 3.9 |
| chloro | 4 | 0 | 0 | 0 | 0.41 | 570 | 4.49 |
| methoxy | 4 | 0 | 4 | 0 | 0.43 | | |
| none | 0 | 0 | 0 | 0 | 0.46 | 550 | 3.28 |
| fluoro | 4 | 0 | 0 | 0 | 0.46 | | |
| methoxy | 2 | 0 | 2 | 4 | 0.5 | | |
| methoxy | 2 | 4 | 0 | 0 | 0.82 | 620 | 8.13 |
| methoxy | 2 | 0 | 4 | 0 | 1.61 | | |
| methoxy | 2 | 3 | 0 | 0 | 3.42 | | |
| methyl | 2 | 0 | 0 | 0 | 3.5 | | |
| fluoro | 2 | 0 | 0 | 0 | 6.2 | | |
| chloro | 2 | 0 | 0 | 0 | 7.3 | 540 | 2.8 |
| bromo | 2 | 0 | 0 | 0 | 7.4 | 560 | 4.07 |
| methoxy | 2 | 0 | 0 | 0 | 7.7 | | |
| chloro | 2 | 4 | 0 | 0 | 17 | 558 | 4.6 |
| chloro | 3 | 0 | 0 | 0 | | 540 | 2.9 |
| chloro | 0 | 0 | 2 | 2 | | 560 | 1.32 |
| nitro | 3 | 0 | 0 | 0 | | 540 | 1.14 |
| nitro | 4 | 0 | 0 | 0 | | 530 | 1.38 |
| cyano | 4 | 0 | 0 | 0 | | 560 | 2.19 |
| -CF₃ | 4 | 0 | 0 | 0 | 3.22 | | |

Herein, the term "hexaaryl bisimidazole" includes salts of the hexaaryl bisimidazole.

Based on the total weight of all polymerizable compounds having at least one ethylenically unsaturated bond used in the light-curable dental material, the hexaaryl bisimidazole may be used in an amount of from 0.01 to 2.0, preferably from 0.05 to 1.0, and most preferably from 0.1 to 0.7 weight-%. It is possible to combine two or more different hexaaryl bisimidazole compounds in the light-curable dental material of the invention. In the latter case, the amounts given apply to the sum of all hexaaryl bisimidazoles used.

The light-curable dental material of the invention generally further comprises a co-initiator selected from the following compound classes: thiols, heteroaromatic thiols, benzothiazoles, benzooxazoles, amines such as tertiary amines, alcohols, thiocarboxylic acids. Specific examples of co-initiators are 3-mercapto-4-methyl-4H-1,2,4-triazole or 2-mercaptobenzimidazole. The co-initiator may be used in an amount of from 0.01 to 2.0, preferably of from 0.05 to 1.5, more preferably 0.05 to 1.0, more preferably from 0.1 to 1.0 and most preferably from 0.1 to 0.7 weight-% based on the total weight of all polymerizable compounds having at least one ethylenically unsaturated bond used in the light-curable dental material. It is possible to combine two or more different co-initiators in the light-curable dental material of the invention. In the latter case, the amounts given apply to the sum of all co-initiators used.

Light sources as commonly used by dentists may be used for polymerizing the light-curable dental materials of the invention, such as those used for polymerizing light-curable dental materials containing camphor quinone as photoinitiator. Such light sources emit cold blue light within the wavelength range of from 400 to 550 nm. A suitable light source is the Spectrum Lite™ from Dentsply.

The light-curable dental material of the invention may also comprise a sensitizer to extend the useful range of absorption wavelengths. Suitable sensitizers include but are not limited to coumarin and derivatives of coumarin such as 7-diethylamino-3-thenoylcoumarin, 7-diethylamino-4-methylcoumarin, 3-Benzoyl-7-methoxycoumarin, 2,3,5,6-1H,4H-tetrahydro-8-methylquinolizino-[9,9A,1GH] coumarin, derivatives of cyclopentanone such as JAW (2,5-Bis[(1H, 5H benzil[I,J]quinolizin-1-yl)methylene]-cyclopentanone), 2,5-benzylidenecyclopentanone, DEAW dye (cyclopentanone-2,5-bis[[4-diethylamino)phenyl]methylene-2E,5E), salts such as bis(4-t-butyl phenyl) iodonium hexafluorophosphate, borate ion, cyanine dyes such as a copper(II) phthalocyanine dye, squarilium and its derivatives, cyclobutenediylium 1,3-bis[(1,3,3-trimethyl-2H-indol-2-ylidene)methyl]-2,4-dihydroxy- bis inner salt, N-phenylglycine, eosin, methylene blue, derivatives of tropinone or tropanone such as tropanone 2,4-bis[2-(3-ethyl-2 (3H)-benzoxazylidene) ethylidene], aryl ketones, and dialkylaminoaldehydes.

The light-curable dental material of the invention generally further contains a polymerization inhibitor. Examples of polymerization inhibitors are phenolic compounds such as BHT or stable radicals such as 2,2,4,4-tetramethylpiperidinyl-1-oxy radical, 2,2-diphenyl-1-picrylhydrazyl radical, galvinoxyl radical, or triphenylmethyl radical. It is possible to combine two or more different polymerization inhibitors in the light-curable dental material of the invention. The amounts of the polymerization inhibitors is chosen such that a useful working time is achieved.

The light-curable dental material may be a dental composite material, a dental glass ionomer, a dental sealant, a dental adhesive, a adhesion promoter, an adhesion preventing material, a cement, a crown-forming material, or an impression material. In an important embodiment, the light-curable dental material is a filler-containing light-curable dental material that contains at least 10 weight-% of a solid particulate filler. Depending on the type of the light-curable dental material, a suitable solvent may be present as generally known in the art. The utility of the bisarylimidazole initiators of the invention does not depend on the particular type or purpose of the light-curable dental material.

The light-curable dental material of the invention contains at least one polymerizable compound having at least one ethylenically unsaturated bond. Said polymerizable compound having at least one ethylenically unsaturated bond may be a polymerizable (meth)acrylic monomer having a (meth)acryl moiety.

In one embodiment, the light-curable dental material contains at least one polymerizable (meth)acrylic monomer having at least two polymerizable groups, such as at least two (meth)acrylic moieties, for allowing cross-linking upon light-curing. Said polymerizable (meth)acrylic monomer having at least two polymerizable groups has at least two (i.e. two, three, four or more) polymerizable groups. Such polymerizable monomers are known to the skilled person from conventional dental materials. Examples are di(meth)acrylates of alkanediols or alkanediamines and other poly- or multifunctional (meth)acrylates; urethane di(meth)acrylates which may be reaction products of 2 mol of a hydroxyalkyl (meth)acrylate with 1 mol of diisocyanate. Specific examples include 1,3-butylene glycol di(meth)acrylate, 1,6-hexanediol di(meth)acrylate, neopentyl glycol di(meth)acrylate, bisphenol A di(meth)acrylate, bisphenol A glycidyl di(meth)acrylate, ethylene oxide-modified bisphenol A di(meth)acrylate, ethylene oxide-modified bisphenol A glycidyl di(meth)acrylate, 2,2-bis(4-methacryloxypropoxyphenyl)-propane, 7,7,9-trimethyl-4,13-dioxa-3,14-dioxo-5,12-diazahexadecane-1,1,6-diol di(meth)acrylate (UDMA), neopentyl glycol hydroxypivalate di(meth)acrylate, caprolactone-modified neopentyl glycol hydroxypivalate di(meth)acrylate, trimethylolethane di(meth)acrylate, trimethylolpropane di(meth)acrylate, and the like. 2, 3 or more different polymerizable monomers may be used as a mixture.

Examples of polymerizable (meth)acrylic monomers having three or more polymerizable groups are trimethylolmethane tri(meth)acrylate, trimethylolethane tri(meth)acrylate, trimethylolpropane tri(meth)acrylate, pentaerythritol tri(meth)acrylate, dipentaerythritol tri(meth)acrylate, pentaerythritol tetra(meth)acrylate, dipentaerythritol tetra(meth)acrylate, dipentaerythritol hexa(meth)acrylate, and the like.

Further examples of polymerizable compounds having at least one ethylenically unsaturated bond are polymerizable monomers like (meth)acrylic monomers having one (meth)acrylic moiety like methyl (meth)acrylate, ethyl (meth)acrylate, n-butyl (meth)acrylate, isobutyl (meth)acrylate, 2-ethylhexyl (meth)acrylate, lauryl (meth)acrylate, tridecyl (meth)acrylate, stearyl (meth)acrylate, cyclohexyl (meth)acrylate, benzyl (meth)acrylate, glycidyl (meth)acrylate, allyl (meth)acrylate, phenoxydiethylene glycol (meth)acrylate, phenoxyhexaethylene glycol (meth)acrylate, dicyclopentenyl (meth)acrylate, isobornyl (meth)acrylate, phenyl (meth)acrylate, caprolactone-modified tetrahydrofurfuryl (meth)acrylate, caprolactone-modified dipentaerythritof (meth)acrylate, caprolactone-modified 2-hydroxyethyl (meth)acrylate, acrylamide and ethylenediamine di(meth)acrylamide.

Examples of polymerizable compounds having at least one ethylenically unsaturated bond other than (meth)acrylic moieties are 1-alkenes, such as 1-hexene, 1-heptene; branched alkenes, such as vinylcyclohexane, 3,3-dimethyl-1-propene, 3-methyl-1-diisobutylene, 4-methyl-1-pentene; vinyl esters, such as vinyl acetate; styrene, substituted styrenes having an alkyl substituent in the side chain, e.g. alpha-methylstyrene, substituted styrenes having an alkyl substituent on the ring, such as vinyltoluene and p-methylstyrene, halogenated styrenes, such as monochlorostyrenes and dichlorostyrenes; or heterocyclic vinyl compounds, such as 2-vinylpyridine, 3-vinylpyridine, 2-methyl-5-vinylpyridine, 3-ethyl-4-vinylpyridine, 2,3-dimethyl-5-vinylpyridine, vinyl-pyrimidine, vinylpiperidine, 9-vinylcarbazole, 3-vinylcarbazole, 4-vinylcarbazole, 1-vinylimidazole, 2-methyl-1-vinylimidazole, N-vinylpyrrolidone, 2-vinyl-pyrrolidone, N-vinylpyrrolidine, 3-vinylpyrrolidine, N-vinylcaprolactam, N-vinylbutyrolactam, vinyloxolane, vinylfuran; vinyl and isoprenyl ethers; maleic acid derivatives, such as maleic anhydride, methylmaleic anhydride, maleimide, methylmaleimide; and dienes, such as divinylbenzene. The polymerizable compounds may be employed as a mixture. They may be used to adjust the mechanical properties of the polymerized light-cured dental material as the case requires.

It is possible to use a conventional photoinitiator such as camphor quinone in addition to the hexaaryl bisimidazole in the light-curable dental material of the invention. In this embodiment, the function of the hexaaryl bisimidazole may be an inhibitor, possibly in addition to the function as a photoinitiator.

In one embodiment, the light-curable dental material is a filler-containing light-curable dental material, such as a dental composite. In this embodiment, the light-curable dental material of the invention comprises a solid particulate filler and a polymerizable matrix, wherein the polymerizable matrix comprises
(i) one or more polymerizable compounds each having at least one ethylenically unsaturated bond
(ii) a hexaaryl bisimidazole, and
(iii) a co-initiator selected from the following compound classes: thiols, heteroaromatic th lois, benzothiazoles, benzooxazoles, tertiary amines, alcohols, and thiocarboxylic acids.

The light-curable dental composite of the invention (or other filler-containing light-curable dental material) contains a solid particulate filler that provides strength to the polymerized dental composite of the invention. The solid filler is a finely divided particulate material. The filler-containing light-curable dental material such as the dental composite contains at least 10% by weight, preferably at least 20% by weight, more preferably at least 50% by weight, and most preferably at least 70% by weight of said solid filler based on the total weight of said filler-containing light-curable dental material. The filler content may be expressed as volume-%, which makes the numerical value of the filler content independent of the density of said solid filler. Using this definition, the filler-containing light-curable dental material such as the dental composite of the invention contains generally at least 4 % by volume, preferably at least 8% by volume, more preferably at least 25 % by volume, even more preferred at least 35% by volume and most preferably at least 45 % by volume of said solid filler. Obviously, the exact amount of said solid filler that can be incorporated into said polymerizable matrix depends on the size of the particles of said solid filler, i.e. on the surface area of said solid filler, and on the density of the filler.

Suitable fillers that may be used in the filler-containing light-curable dental material, notably the light-curable dental composite, include organic and inorganic solid fillers, whereby inorganic fillers are preferred. Examples of inorganic fillers are glasses e.g. those containing barium, strontium, boron, or zinc, aluminosilicate glass, and metal oxides such as zinc oxide, zirconium oxide, aluminium oxide, silica, apatite, or a cured mixture of resin and filler ground or otherwise reduced in size to a powder. Other examples are fused silica, quartz, crystalline silica, amorphous silica, soda glass beads, glass rods, ceramic oxides, particulate silicate glass, radiopaque glasses (barium and strontium glasses), and synthetic minerals. It is also possible to employ finely divided materials and powdered hydroxyl-apatite, although materials that react with silane coupling agents are preferred. Also available as a filler are colloidal or submicron silicas coated with a polymer. As further examples of suitable inorganic fillers may be mentioned La₂O₃, ZrO₂, BiPO₄, CaWO₄, BaWO₄, SrF₂, Bi₂O₃. Suitable organic fillers include polymer granulates such as polytetrafluoroethylene particles. Small amounts of pigments to allow matching of the composition to various shades of teeth can be included.

The particles of said solid filler should have a mean size below 100 µm, preferably below 50 µm, more preferably below 20 µm. Two or more solid fillers may be mixed that differ in their mean particle size. The particle size distribution may be monomodal or may be polymodal. Preferably, the particle size distribution is bimodal, e.g. as described in WO 2000/61073. The particles may be of any desired shape, for instance spherical, irregular as is obtained by mechanical particle size reduction, fibres, whiskers, platelets, dumbbell shaped, or cylindrical, and may be solid, hollow, or porous. Solid fillers that may be used in the present invention are known in the art. Inorganic fillers are preferably silanated before use in the present invention to render the surface of the filler particles more hydrophobic. Silanating agents for this purpose are well known in the art, e.g. 3-methacryloxypropyltrimethoxysilane. Dental materials containing solid fillers of different particle sizes are for example described in WO 00/61073.

The polymerizable matrix of the light-curable dental composite of the invention may further comprise from 1.0 to 50, preferably from 1.0 to 15 weight-% of at least one polymerizable monomer having a carboxylic acid group based on the total weight of said polymerizable matrix, said polymerizable monomer having a carboxylic acid group. Regarding said polymerizable monomer having a carboxylic acid group and the amounts of it to be used in the polymerizable matrix of the light-curable dental composite, reference is made to WO 2006/084769 that is included herein by reference in its entirety.

The light-curable dental composite typically further contains a suitable co-initiator and an inhibitor as described above.

In another embodiment, the filler-containing light-curable dental material is a dental glass ionomer cement. Ionomer cements commonly contain a polycarboxylic acid and an inorganic powder which reacts in the presence of water by a curing reaction. Conventional ionomer cements generally contain a powder component containing aluminosilicate and a liquid portion usually containing a polyacid such as polyacrylic acid, polymaleic acid, polyitaconic acid, or a copolymer of at least two of the acids, cf. "New Aspects of the Setting of Glass- ionomer Cements," Wasson et al., Journal of Dental Research; Vol. 72, No. 2, February, 1993; pages 481-483. In glass ionomer cements, the primary reactions which cause the glass ionomer cement to harden is cross-linking of polycarboxylate chains by metal ions from the glass based on ionic forces. Moreover, during setting the acids of the glass ionomer cement dissolve the glass structure to release metal constituents of the glass. Ionic carboxylates of calcium, strontium and aluminum are mainly formed during the setting process. In the present invention, the polymerizable matrix of the light-curable dental ionomer cement contains said one or more polymerizable compounds each having at least one ethylenically unsaturated bond in addition to the polycarboxylic acid, and the hexaarylbisimidazole of the invention.

For dental ionomer cements, the fillers are particulate reactive fillers. A "particulate reactive filler" is a powdered metal oxide or hydroxide, mineral silicate, or ion leachable glass or ceramic, that is capable of reacting with an ionomer in the presence of water to form a hydrogel. Examples of particulate reactive filler materials include materials commonly known in the art of glass-ionomer cements such as calcium or strontium-containing and aluminum-containing materials. Preferably, particulate reactive fillers contain leachable fluoride ions. Specific examples of particulate reactive fillers are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass. The glasses may also contain other elements such as zinc, lanthanum, silver, copper, and iron. Suitable particulate reactive fillers further include metal oxides such as zinc oxide and magnesium oxide, and ion-leachable glasses, e.g., as described in US- A 3,655,605, US-A 3,814,717, US-A 4,143,018, US-A 4,209,434, US-A 4,360,605, US-A 4,376,835 and W02005EP11584A.

The particulate reactive filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus. The particulate reactive filler may be a multimodal particulate reactive filler representing a mixture of two or more particulate fractions having different average particle sizes. The particulate reactive filler may also be a mixture of particles of different chemical composition. In particular, it is possible to use a mixture of a particulate reactive material and a particulate non-reactive material.

The light-curable dental ionomer cement typically further contains a suitable co-initiator and an inhibitor as described above.

### Experimental part

### Measurement of compressive and yield strengths

Metal forms with an internal diameter of 4 mm and a height of 6 mm as described in ISO 9917 section 7.4 were used to prepare the specimens. The paste to be measured was filled into the forms, covered with polyester foil, and pressed with metal plates to extrude excess material. The material was then cured for 40 seconds from each end using a dental curing lamp (Spectrum Lite, Dentsply) with an output between 600 and 700 mW/cm². The forms complete with specimen were drawn across silicon carbide paper (600 grit) until a smooth surface level with the end of the form was obtained, and then the cured specimens were removed from the form. The specimens were stored in water at 37°C for 24 hours before being tested in a universal testing machine (Zwick) with a crosshead speed of 1 mm/minute. Compressive strength was measured by loading the specimens to failure, and recording the maximum force reached divided by the cross section area of the specimen. The stress strain curve for each specimen was inspected and found to consist essentially of an initial straight portion followed by a curved portion leading to the final breaking point. The straight portion of the curve corresponds to elastic behaviour of the material, whereas the curved portion corresponds to plastic flow. The force at which the stress strain curve first deviated from a straight line was taken as the yield point. The yield point is expressed in MPa, and is calculated by dividing the yield force in Newtons by the cross-sectional area of the specimen. The average value of at least five specimens for each material was calculated.

### Measurement of flexural strength

Glass tubes with internal diameter 4 mm and length about 30 mm where filled with the composite material to a length of about 25 mm. The tubes were placed in a LiCu Lite light oven (Dentsply) and hardened by exposure to the light for two minutes. After this the resulting hardened composite cylinders were pushed from the tubes and stored in water at 37°C for 24 hours. The flexural strength was measured by testing the specimens to failure in three-point bending mode using a Zwick universal testing machine. The average value of at least five specimens for each material was calculated.

### Measurement of Vickers hardness (VH)

The composite material was filled into forms 8 mm diameter and 2 mm thick. The composite was hardened for 20 seconds with a dental curing lamp (Spectrum Lite, Dentsply) with an output between 600 and 700 mW/cm². After this the specimens were removed from the forms and stored in water at 37°C for 24 hours before being measured using a Vickers Hardness apparatus (Frank).

### Lifetime and depth of cure

The lifetime (sensitivity to ambient light) of the materials was measured at 10000 lux using the method given in ISO 4049:2000. Note however that ISO 4049:2000 specifies a lower light intensity of 8000 ± 1000 lux. The depth of cure of the materials was also measured according to the method given in ISO 4049:2000.
1. Synthesis of bis 2-(2-chlorophenyl-4,5-diphenylimidazole), (2Cl-HABI)
   1.1. The precursor 2-(2-chlorophenyl-4,5-diphenylimidazole) was prepared according to a literature method (e.g. US 3,784,557). Benzil (21 g) and ammonium acetate (60 g) were added to glacial acetic acid (500 ml) and the mixture was stirred to dissolve the benzil. 2-chlorobenzaldehyde (14 g) was then added and the mixture was stirred and heated to 125°C for 2 hours. The mixture was allowed to cool and was then poured into water (1800 ml) with stirring to give a pale yellow viscous mass. This gradually solidified to give a pale yellow solid which was filtered and dried (39 g). The solid was dissolved in boiling ethanol (400 ml) and after cooling, filtering, and drying gave an off-white slightly yellow fluorescing powder (18.3 g).
   1.2. Bis 2-(2-chlorophenyl-4,5-diphenylimidazole) was prepared from the product of 1.1 by oxidation with aqueous potassium ferricyanide, as described in J. Org. Chem 36, 16, 1971, 2262-2266. The product from 1.1 (4.95 g) was slurried with toluene (150 ml). A solution of potassium ferricyanide (9.9 g), and sodium hydroxide (6.0 g) in water (100 ml) was added to the toluene slurry, and the two phases stirred vigorously together. The solid dissolved over about one hour after which a mauve organic phase had been formed. The mixture was stirred together for about 18 hours after which the organic layer was separated and dried over anhydrous MgSO₄. The organic layer was a yellow colour when stored in the dark, but turned mauve after a short exposure to light. The toluene was removed under vacuum to give a viscous yellow liquid. Diethyl ether (50 ml) was added and precipitation initiated by scratching inside the flask with a glass rod. After 2 hours the ether was decanted to leave yellow crystals (4.6 g). H¹NMR analysis showed the crystals to be an approximately 1:1 complex between ether and bis 2-(2-chlorophenyl-4,5-diphenylimidazole). The crystals were stored at 40°C under vacuum after which pure bis 2-(2-chlorophenyl-4,5-diphenylimidazole) was obtained (4.44 g).
   2. Synthesis of Bis 2-(2,4-dichlorophenyl-4,5-diphenylimidazole), (2,4-Cl HABI) 2-(2,4-dichlorophenyl-4,5-diphenylimidazole) and bis 2-(2,4-dichlorophenyl-4,5-diphenylimidazole) were synthesised analogously to the methods in 1 above by substituting the 2-chlorobenzaldehyde by a molar equivalent of 2,4-dichlorobenzaldehyde. Benzil (21 g) and ammonium acetate (60 g) were dissolved in glacial acetic acid (400 ml). 2,4-dichlorobenzaldehyde was then added and the mixture stirred and heated to reflux for 2 hours. After cooling, the mixture was poured into cold water (1500 ml) with vigorous stirring. The white precipitate was filtered off and dried in vacuum to give 97 g. This was refluxed with a mixture of ethanol (350 ml) and acetic acid (150 ml), filtered hot, and then allowed to cool. The white crystals were filtered off and dried to give (21.1 g) of 2-(2,4-dichlorophenyl-4,5-diphenylimidazole). Water was slowly added to the filtrate until this just turned cloudy. After cooling in a refrigerator, a further 6.6 g of crystals were obtained. The 2-(2,4-dichlorophenyl-4,5-diphenylimidazole) was oxidised to the dimer bis 2-(2,4-dichlorophenyl-4,5-diphenylimidazole) following the procedure given in method 1.2 for bis 2-(2-chlorophenyl-4,5-diphenylimidazole), except that the toluene was replaced by dichloromethane as solvent.
   3. Synthesis of Bis(2,4,5-triphenylimidazole), also known as Lophine dimer, or HABI 2,4,5-triphenylimidazole and bis(2,4,5-triphenylimidazole) were prepared analogously to the method in 2 above, by substituting the 2,4-dichlorobenzaldehyde by a molar equivalent of benzaldehyde.
   4. 2-(2-methoxyphenyl-4,5-diphenylimidazole) and bis Bis 2-(2-methoxyphenyl-4,5-diphenylimidazole) were synthesised analogously to the method in 2 by substituting the 2,4-dichlorobenzaldehyde for a molar equivalent of 2-methoxybenzaldehyde.
   5. Further, differently substituted hexaarylbisimidazoles were synthesised analogously to the method in 1 by exchanging the 2-chlorobenzaldehyde by a molar equivalent of the appropriately substituted aldehyde, for example 4-methoxybenzaldehyde, 2,4-dimethoxybenzaldehyde, 4-cyanobenzaldehyde, naphthaldehyde, 2-trifluoromethyl benzaldehyde, and the like.
   6. Yet further substituted hexaarylbisimidazoles were synthesised analogously to the method in 1 by exchanging benzil by a molar equivalent of the appropriately substituted benzil, for example 4,4'-dimethoxybenzil, 4,4'-dimethylbenzil, 2-chloro-3',4'-dimethoxybenzil, and the like. The same approach may be taken for preparing hexaarylbisimidazoles having one or more heteroaromatic aryl groups.

### Examples 1 to 23

Preparation of experimental resin mixtures: Resin mixtures were prepared with initiator and co-initiator concentrations as given in Table 2 by first mixing together UDMA (40 parts), HPGM (7 parts), and EBA (53 parts) at 40°C for Resin A, or UDMA (5 parts), HPGM (5 parts), TMPTMA (5 parts), and EBA (85 parts) for Resin B. The initiators and inhibitors were then added as required in amounts shown in the table, and dissolved by stirring at 40 - 50°C until no solid particles remained.

Preparation of dental composites: The chosen resin mixture (33.0 g), Aerosil R972 (0.75 g), and silanated glass with a mean particle size of about 0.8 µm (116.25 g) were kneaded together at 40°C in a vertical kneader for 160 minutes, and the resulting paste was then degassed by stirring for ten minutes at a pressure of 210±10 mbar. Physical properties of the pastes were measured as described below, and are shown in Table 2.

**Table 2: Formulations comprising hexaarylbisimidazoles, with measured physical properties**

| Ex. No. | resin | initiator | initiator | co-initiator | %co-initiator | BHT | comp. str. | yield str. | flex. str. | VH5 | life-time | Doc |
|---|---|---|---|---|---|---|---|---|---|---|---|---|
| | | - | % | - | % | % | MPa | MPa | MPa | | sec | mm |
| 1 | A | 2-Cl HABI | 0.5 | MMT | 0.5 | 0.0 | 327.5 | 152 | 139 | 67 | 300 | 2.0 |
| 2 | A | 2-Cl HABI | 0.5 | MMT | 0.25 | 0.0 | 325.6 | 150 | 138.5 | 60 | 480 | 1.8 |
| 3 | A | 2-Cl HABI | 0.25 | MMT | 0.5 | 0.0 | 302.9 | 136 | 131.5 | 54 | 540 | 1.8 |
| 4 | A | 2-Cl HABI | 0.25 | MMT | 0.25 | 0.0 | 284.6 | 124 | 141.4 | 55 | 600 | 1.6 |
| 5 | A | 2-Cl HABI | 0.75 | MMT | 0.25 | 0.0 | 348.5 | 163 | 138.7 | 63 | 360 | 1.9 |
| 6 | A | 2,4-Cl HABI | 0.5 | MMT | 0.5 | 0.0 | 322.0 | 175 | 142.5 | 59 | 180 | 2.0 |
| 7 | A | 2,4-Cl HABI | 0.5 | MMT | 0.25 | 0.0 | 283.5 | 152 | 141.1 | 57 | 420 | 3.0 |
| 8 | A | 2,4-Cl HABI | 0.25 | MMT | 0.5 | 0.0 | 260.6 | 131 | 122.0 | 49 | 480 | 2.0 |
| 9 | B | 2-Cl HABI | 0.5 | MMT | 0.5 | 0.05 | 253.2 | 110 | 93.0 | 23 | >300 | 1.6 |
| 10 | B | TCDM-HABI | 0.5 | MMT | 0.5 | 0.05 | 280.9 | - | 89.1 | - | >300 | 1.8 |
| 11 | B | 2,4-Cl HABI | 0.5 | MMT | 0.5 | 0.05 | 267.2 | 127 | 124.6 | - | >300 | 2.0 |
| 12 | B | 2,4-Cl HABI | 0.5 | MMT | 0.75 | 0.05 | 317.5 | 156 | 150.2 | 57 | >300 | 2.3 |
| 13 | B | 2-Cl HABI | 0.66 | MMT | 0.35 | 0.05 | 272.4 | 131 | 132.6 | 41 | >300 | 2.0 |
| 14 | B | TCDM-HABI | 0.75 | MMT | 0.35 | 0.05 | 271.5 | 120 | 122.4 | 28 | >300 | 1.9 |
| 15 | B | 2,4-Cl HABI | 0.73 | MMT | 0.35 | 0.05 | 277.0 | 138 | 140.0 | 47 | >300 | 2.24 |
| 16 | B | 2-TFM HABI | 0.73 | MMT | 0.35 | 0.05 | 281.5 | 135 | 137.9 | 33 | >300 | 2.06 |
| 17 | B | 2-Cl HABI | 0.66 | MBT | 0.51 | 0.05 | 299.4 | 152 | 123.2 | 55 | >300 | 2.3 |
| 18 | B | TCDM-HABI | 0.75 | MBT | 0.51 | 0.05 | 208.5 | - | - | 54 | >300 | 1.6 |
| 19 | B | 2,4-Cl HABI | 0.73 | MBT | 0.51 | 0.05 | 297.3 | 149 | 122.1 | 59 | >300 | 2.3 |
| 20 | B | 2-TFM HABI | 0.73 | MBT | 0.51 | 0.05 | 293.9 | 144 | 144.9 | 54 | >300 | 2.1 |
| 21 | B | 2,4-Cl, HABI | 0.73 | MBT | 0.76 | 0.05 | 319.6 | 160 | 150.7 | 58 | 280 | 3.4 |
| 22 | B | 2-TFM HABI | 0.73 | MBT | 0.76 | 0.05 | 318.2 | 156 | 161.9 | 59 | 210 | 2.5 |
| 23 | B | 2,4Cl HABI / CQ | 0.73 / 0.31 | DMABE | 0.6 | 0.3 | 284.4 | 136 | 127 | 49 | >300 | - |

### Comparative examples

| No. | Resin | Initiator | initiator | co initiator | % co-initiator | BHT | comp. str. | yield str. | flex. str. | VH5 | life-time | Doc |
|---|---|---|---|---|---|---|---|---|---|---|---|---|
| | | - | % | - | % | % | MPa | MPa | MPa | | sec | mm |
| 1 | A | CQ | 0.31 | DMABE | 0.6 | 0.6 | 293.5 | 133 | 119.7 | 58 | 135 | 3.0 |
| 2 | B | CQ | 0.31 | DMABE | 0.6 | 0.6 | 264.8 | 145 | 100.7 | 58 | 70 | 3.6 |

### Key to abbreviations:

| | |
|---|---|
| UDMA | Urethane dimethacrylate |
| HPGM | the reaction product of Hydroxypropyl methacrylate and glutaric anhydride |
| EBA | Ethoxylated bisphenol A dimethacrylate |
| CQ | Camphorquinone |
| DMABE | Dimethylamino benzoic acid, ethyl ester |
| BHT | butylated hydroxytoluene |
| MMT | 3-Mercapto-4-methyl-4H-1,2,4-triazole |
| 2-Cl HABI | 2,2'-Bis(2-chlorophenyl)-4,4',5,5'-tetraphenyl-1,1'-biimidazole |
| 2,4-Cl HABI | 2,2'-Bis(2,4-dichlorophenyl)-4,4', 5,5'-tetraphenyl-1,1'-biimidazole |
| TCDM | 2,2,'4-Tris(2-chlorophenyl)-5-(3,4-dimethoxyphenyl)-4',5'-diphenyl-1,1'-biimidazole |
| 2-TFM HABI | 2,2'-Bis(2-trifluoromethyl)-4,4',5,5'-tetraphenyl-1,1'-biimidazole |
| CDM | 2-(o-Chloro)-4,5-bis(m-methoxyphenyl)-1,1'-biimidazole |
| TCTM | 2,5-Bis(o-chlorophenyl)-4-[3,4-dimethoxyphenyl]-1 H-imidazole dimer |
| 2-MBO | 2-Mercaptobenzoxazole |
| 2-MBT | 2-Mercaptobenzothiazole |
| Doc | depth of cure |
| VH5 | Vickers hardness measured with a load of 5 kg (49.03 N) |

It is seen from the examples in Table 2 that the use of a bisimidazole initiator according to the present invention leads to lengthened lifetimes of the compositions under the influence of ambient light at 10000 lux. Further, composite material with improved compressive, yield, and flexural strengths compared to the comparative examples can be obtained.

## Claims

1. A light-curable dental material comprising
(i) a polymerizable compound having at least one ethylenically unsaturated bond,
(ii) a hexaaryl bisimidazole, and
(iii) a co-initiator selected from the following compound classes: thiols, heteroaromatic thiols, benzothiazoles, benzooxazoles, tertiary amines, alcohols, and thiocarboxylic acids.

2. The light-curable dental material according to claim 1 , further comprising an α,β- diketone as a polymerization initiator and optionally a polymerisation inhibitor.

3. The light-curable dental material according to any one of claims 1 or 2. further comprising a solid particulate filler.

4. The light-curable dental material according to any of claims 1 to 3, wherein said hexaaryl biimidazole has the following structure (I):
Ar₃T-TAr₃ (I)
wherein each T is an imidazole moiety and each Ar stands for an optionally substituted aryl group that may be the same or different and wherein the substitution on different groups Ar may be the same or different.

5. The light-curable dental material according to claim 1 or 4, wherein the hexaaryl bisimidazole is a homodimer or a heterodimer obtainable by oxidation of any of the triarylimidazoles represented by the following formula (II): 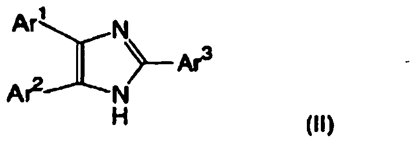 wherein Ar¹, Ar², and Ar³ may be the same or different and each represents an optionally substituted aryl group and multiple Ar¹, Ar², or Ar³ on the same hexaarylbisimidazole may be the same or different.

6. The light-curable dental material according to any one of claims 1 to 5, wherein the hexaaryl bisimidazole has the structure of the following formula (III): 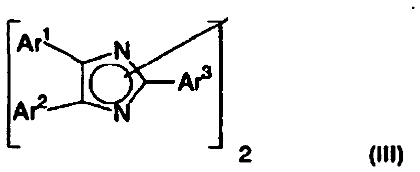 wherein Ar¹, Ar², and Ar³ may be the same or different and each represents an optionally substituted aryl group and multiple Ar¹, Ar², or Ar³ on the same hexaarylbisimidazole compound may be the same or different.

7. The light-curable dental material according to any one of claims 4 to 6, wherein the aryl group is selected from phenyl and naphthyl.

8. The light-curable dental material according to any one of claim 4 to 7, wherein each aryl group may have 1 to 5 substituents selected from the group consisting of C₁₋₆ alkyl, C₁₋₆ alkoxy, hydroxy, halo, cyano, nitro, nitroso, mercapto, carboxyl, sulfonate, thiol, amino, phenyl, pyridyl, and trifluoromethyl.

9. The light-curable dental material according to any one of claim 1 to 8, wherein the concentration of said hexaaryl bisimidazole based on the total weight of all polymerizable compounds having at least one ethylenically unsaturated bond is from 0.01 to 2.0, preferably from 0.05 to 1.5, and most prefeably from 0.1 to 1.0 weight-%.

10. The light-curable dental material according to any one of claim 1 to 9, further comprising a sensitizer for extending the wavelength range usable for activating the hexaaryl bisimidazole.

11. The light-curable dental material according to any one of claims 1 to 10 , wherein the light-curable dental material is selected from the group consisting of a dental composite, dental ionomer cement, dental sealant, dental adhesive, dental adhesion promoter, dental adhesion preventer, dental cement, dental crown-forming material, and dental impression material.

12. Light-cured dental material obtained or obtainable by light curing the light-curable dental material of any one of claims 1 to 11.

13. Use of a hexaaryl bisimidazole as a photoinitiator in a light-curable dental material as defined in any one of claims 1 to 11.

## Patentansprüche

1. Lichthärtendes Dentalmaterial, umfassend
(i) eine polymerisierbare Verbindung mit mindestens einer ethylenisch ungesättigten Bindung,
(ii) ein Hexaarylbisimidazol, und
(iii) einen Coinitiator, ausgewählt aus den folgenden Verbindungsklassen: Thiole, heteroaromatische Thiole, Benzothiazole, Benzooxazole, tertiäre Amine, Alkohole und Thiocarbonsäuren.

2. Das lichthärtende Dentalmaterial nach Anspruch 1, ferner umfassend ein α,β-Diketon als Polymerisationsinitiator und gegebenenfalls einen Polymerisationsinhibitor.

3. Das lichthärtende Dentalmaterial nach einem der Ansprüche 1 oder 2, ferner umfassend einen festen teilchenförmigen Füllstoff.

4. Das lichthärtende Dentalmaterial nach einem der Ansprüche 1 bis 3, wobei das Haxaarylbisimidazol die folgende Sturktur (I) aufweist:
Ar₃T-TAr₃ (I)
wobei jedes T eine Imidazoleinheit bedeutet und jedes Ar für eine gegebenenfalls substituierte Arylgruppe steht, die gleich oder verschieden sein können, und wobei die Substitution auf verschiedenen Ar-Gruppen gleich oder verschieden sein kann.

5. Das lichthärtende Dentalmaterial nach Anspruch 1 oder 4, wobei das Hexaarylbisimidazol ein Homodimer oder Heterodimer ist, das erhältlich ist durch Oxidation eines Triarylimidazols der folgenden Formel (II): 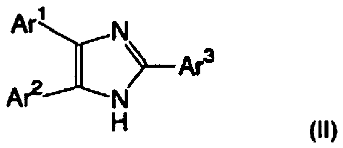 wobei Ar¹, Ar² und Ar³, die gleich oder verschieden sein können, jeweils für eine gegebenenfalls substituierte Arylgruppe stehen, und mehrere Ar¹, Ar² oder Ar³ auf demselben Hexaarylbisimidazol gleich oder verschieden sein können.

6. Das lichthärtende Dentalmaterial nach einem der Ansprüche 1 bis 5, wobei das Hexaarylbisimidazol die Struktur der folgenden Formel (III) aufweist: 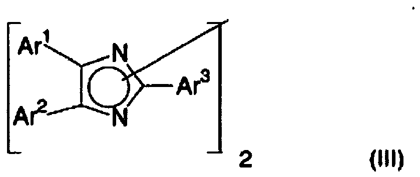 wobei Ar¹, Ar² und Ar³ die gleich oder verschieden sein können und jeweils für eine gegebenenfalls substituierte Arylgruppe stehen und mehrere Ar¹, Ar² oder Ar³ auf der selben Hexaarylbisimidazolverbindung können gleich oder verschieden sein.

7. Das lichthärtende Dentalmaterial nach einem der Ansprüche 4 bis 6, wobei die Arylgruppe ausgewählt wird aus Phenyl und Naphthyl.

8. Das lichthärtende Dentalmaterial nach einem der Ansprüche 4 bis 7, wobei jede Arylgruppe 1 bis 5 Subsituenten aufweisen kann, die ausgewählt werden aus der Gruppe, bestehend aus C₁₋₆Alkyl, C₁₋₆Alkoxy, Hydroxy, Halogen, Cyano, Nitro, Nitroso, Mercapto, Carboxyl, Sulfonat, Thiol, Amino, Phenyl, Pyridyl und Trifluormethyl.

9. Das lichthärtende Dentalmaterial nach einem der Ansprüche 1 bis 8, wobei die Konzentration des Hexaarylbisimidazols, bezogen auf das Gesamtgewicht aller polymerisierbaren Verbindungen mit mindestens einer ethylenisch ungesättigten Bindung, bei 0,01 bis 2,0, vorzugsweise bei 0,05 bis 1,5, und am meisten bevorzugt 0,1 bis 1,0 Gewichts-%, liegt.

10. Das lichthärtende Dentalmaterial nach einem der Ansprüche 1 bis 9, ferner umfassend einen Sensibilisator zur Erweiterung des Wellenlängenbereichs, der zur Aktivierung des Hexaarylbisimidazols brauchbar ist.

11. Das lichthärtende Dentalmaterial nach einem der Ansprüche 1 bis 10, wobei das lichthärtbare Dentalmaterial ausgewählt wird aus der Gruppe, bestehend aus einem Dentalkomposit, Dentalionomerzement, Dentalversiegeler, Dentaladhäsiv, Dentaladhäsion-fördernden Mittel, Dentaladhäsion-verhindernden Mittel, Dentalzement, kronenbildendes Dentalmaterial und dentales Abdruckmaterial.

12. Lichthärtendes Dentalmaterial, erhältlich oder erhalten durch Lichthärtung des lichthärtenden Dentalmaterials aus einem der Ansprüche 1 bis 11.

13. Verwendung eines Hexaarylbisimidazols als Photoinitiator in einem lichthärtenden Dentalmaterial, wie es in einem der Ansprüche 1 bis 11 definiert wird.

## Revendications

1. Matériau dentaire photodurcissable, comprenant
(i) un composé polymérisable ayant au moins une liaison d'insaturation éthylénique,
(ii) un hexaaryl-bis-imidazole, et
(iii) un co-initiateur choisi dans les catégories de composés suivantes : des thiols, des thiols hétéroaromatiques, des benzothiazoles, des benzooxazoles, des amines tertiaires, des alcools et des acides thiocarboxyliques.

2. Matériau dentaire photodurcissable suivant la revendication 1, comprenant en outre une α,β-dicétone comme initiateur de polymérisation et facultativement un inhibiteur de polymérisation.

3. Matériau dentaire photodurcissable suivant l'une quelconque des revendications 1 et 2, comprenant en outre une charge en particules solides.

4. Matériau dentaire photodurcissable suivant l'une quelconque des revendications 1 à 3, dans lequel ledit hexaaryl-bis-imidazole a la structure (I) suivante :
Ar₃T-TAr₃ (I)
dans laquelle chaque T représente un groupement imidazole et chaque Ar représente un groupe aryle, facultativement substitué, qui peuvent être identiques ou différents, et dans laquelle la substitution sur des groupes Ar différents peut être identique ou différente.

5. Matériau dentaire photodurcissable suivant la revendication 1 ou 4, dans lequel l'hexaaryl-bis-imidazole est un homodimère ou un hétérodimère pouvant être obtenu par oxydation de n'importe lequel des triarylimidazoles représentés par la formule (II) suivants : 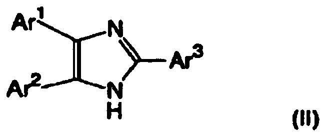 dans laquelle Ar¹, Ar² et Ar³ peuvent être identiques ou différents et représentent chacun un groupe aryle facultativement substitué et des groupes Ar¹, Ar² ou Ar³ multiples sur le même hexaaryl-bis-imidazole peuvent être identiques ou différents.

6. Matériau dentaire photodurcissable suivant l'une quelconque des revendications 1 à 5, dans lequel l'hexaaryl-bis-imidazole a la structure répondant à la formule (III) suivants : 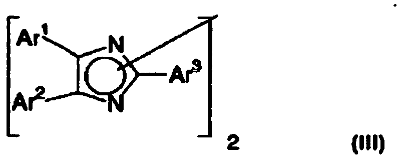 dans laquelle Ar¹, Ar² et Ar³ peuvent être identiques ou différents et représentent chacun un groupe aryle facultativement substitué et des groupes Ar¹, Ar² ou Ar³ multiples sur le même hexaaryl-bis-imidazole peuvent être identiques ou différents.

7. Matériau dentaire photodurcissable suivant l'une quelconque des revendications 4 à 6, dans lequel le groupe aryle est choisi entre les groupes phényle et naphtyle.

8. Matériau dentaire photodurcissable suivant l'une quelconque des revendications 4 à 7, dans lequel chaque groupe aryle peut porter 1 à 5 substituants choisis dans le groupe consistant en des substituants C₁₋₆alkyle, C₁₋₆alkoxy, hydroxy, halogéno, cyano, nitro, nitroso, mercapto, carboxyle, sulfonate, thiol, amino, phényle, pyridinyle et trifluorométhyle.

9. Matériau dentaire photodurcissable suivant l'une quelconque des revendications 1 à 8, dans lequel la concentration dudit hexaaryl-bis-imidazole, sur la base du poids total de tous les composés polymérisables ayant au moins une liaison d'insaturation éthylénique va de 0,01 à 2,0, avantageusement de 0,05 à 1,5 et de préférence de 0,1 à 1,0 % en poids.

10. Matériau dentaire photodurcissable suivant l'une quelconque des revendications 1 à 9, comprenant en outre un sensibilisant pour étendre la plage de longueurs d'ondes utilisable pour activer de l'hexaaryl-bis-imidazole.

11. Matériau dentaire photodurcissable suivant l'une quelconque des revendications 1 à 10, ledit matériau dentaire photodurcissable étant choisi dans le groupe consistant en un composite dentaire, un ciment ionomère dentaire, un agent de scellement dentaire, un adhésif dentaire, un activateur d'adhérence dentaire, un inhibiteur d'adhérence dentaire, un ciment dentaire, un matériau de formation de couronne dentaire et une matériau d'impression dentaire.

12. Matériau dentaire photodurci obtenu ou pouvant être obtenu par photodurcissement du matériau dentaire photodurcissable de l'une quelconque des revendications 1 à 11.

13. Utilisation d'un hexaaryl-bis-imidazole comme photo-initiateur dans un matériau dentaire photodurcissable tel que défini dans l'une quelconque des revendications 1 à 11.
